(19) Europäisches Patentamt
European Patent Office
Office européen des brevets

(11) Veröffentlichungsnummer: **0 133 318**
**B1**

# (12) EUROPÄISCHE PATENTSCHRIFT

(45) Veröffentlichungstag der Patentschrift: **07.01.87**

(51) Int. Cl.⁴: **C 07 C 143/21**

(21) Anmeldenummer: **84108954.3**

(22) Anmeldetag: **27.07.84**

(54) **Cycloaliphatische Aminosulfonsäurederivate, Verfahren zu deren Herstellung und Arzneimittel zur Bekämpfung von Herz- und Gefässkrankheiten.**

(30) Priorität: **29.07.83 DE 3327318**

(43) Veröffentlichungstag der Anmeldung: **20.02.85 Patentblatt 85/8**

(45) Bekanntmachung des Hinweises auf die Patenterteilung: **07.01.87 Patentblatt 87/2**

(84) Benannte Vertragsstaaten: **AT CH DE FR GB IT LI**

(56) Entgegenhaltungen:
**Dr. OTTO-ALBRECHT NEUMÜLLER, "Römpps Chemie-Lexikon", 7.Auflage, Band 5, 1975, Franckh'sche Verlagshandlung, Stuttgart, Seite 2804**

(73) Patentinhaber: **GÖDECKE AKTIENGESELLSCHAFT, Salzufer 16, D-1000 Berlin 10 (DE)**

(72) Erfinder: **Satzinger, Gerhard, Dr., Im Mattenbühl 7, D-7809 Denzlingen (DE)**
Erfinder: **Hartenstein, Johannes, Dr., Fohrenbühl 23, D-7801 Stegen-Wittental (DE)**
Erfinder: **Fritschi, Edgar, Dr., Am Scheuerwald 2, D-7811 St. Peter (DE)**
Erfinder: **Vigelius, Wolf-Dieter, Dr., Berliner Strasse 13/4, D-7809 Denzlingen (DE)**

**EP 0 133 318 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind neue cycloaliphatische Aminosulfonsäurederivate der allgemeinen Formel I

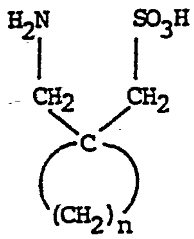

(I)

in welcher n die Zahlen 4, 5 oder 6 bedeutet.

Es wurde gefunden, dass die Verbindungen der allgemeinen Formel I bei sehr guter Verträglichkeit wertvolle cardiovaskuläre Eigenschaften aufweisen und sich insbesondere zur Therapie unerwünschter Reizbildung und Reizleitung im Gewebe, wie sie zum Beispiel bei Herzrhythmusstörungen auftreten, eignen.

Das Mittel der Wahl zur Behandlung der genannten Störungen ist nach wie vor Procainamid, als Prototyp der direkt wirkenden Antiarrhythmika. Procainamid hat aber den schwerwiegenden Nachteil, dass durch seine Anwendung *alle* Basisfunktionen des Herzens reduziert werden. Die erforderliche hohe Behandlungsdosis und eine geringe therapeutische Breite, sowie ausgesprochen unangenehme Begleiterscheinungen, wie Kollaps- und Schockgefahr, Tachykardie, Thrombopenie, Agranulocytose und gastrointestinale und allergische Erscheinungen lassen die Suche nach chemisch neuartigen Substanzen, welche die genannten Nachteile nicht aufweisen, dringend notwendig erscheinen [hierzu: Pharmazie. *34*, S. 494-498 (1979)].

Die neuen Verbindungen der allgemeinen Formel I bedeuten daher einen grossen Fortschritt in der angegebenen Richtung.

Die Verbindungen der allgemeinen Formel I können dadurch hergestellt werden, dass man eine Verbindung der allgemeinen Formel II

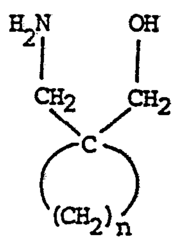

(II)

in welcher n die obengenannte Bedeutung hat, in an sich bekannter Weise zunächst in einen reaktiven Ester überführt und diesen mit einem löslichen anorganischen Sulfit, insbesondere einem Alkalisulfit umsetzt.

Die Verbindungen der allgemeinen Formel II sind bekannt (Bull. Soc. Chim. Fr. 1965. S. 204-208) oder sind nach bekannten Methoden herstellbar.

Als reaktive Ester kommen solche starker anorganischer oder organischer Säuren infrage. Besonders geeignet sind Halogenide und Schwefelsäureester. Besonders bevorzugt werden die Schwefelsäurehalbester der allgemeinen Formel III

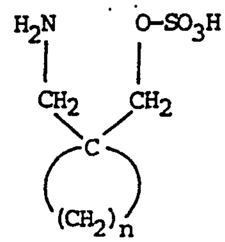

(III)

in welcher n die obengenannte Bedeutung hat.

Auch bei den Verbindungen III handelt es sich um bekannte Verbindungen (Bull. Soc. Chim. France loc. cit).

Die Überführung der Verbindungen der allgemeinen Formel III in die Aminosulfonsäuren der allgemeinen Formel I erfolgt durch Substitution des als Abgangsgruppe fungierenden Restes $-O-SO_3H$ durch Sulfit. Die Reaktion wird zweckmässig so durchgeführt, dass man die Verbindung III mit einem Alkalisulfit, z.B. Natrium- oder Ammoniumsulfit in der Hitze und in einem geeigneten Lösungsmittel umsetzt. Vorzugsweise erhitzt man die Schwefelsäurehalbester der allgemeinen Formel III in Wasser mit einer äquivalenten oder überschüssigen Menge an Natriumsulfit auf Temperaturen von 100-180° C, vorzugsweise 160° C im Autoklaven. Die Umsetzung anderer reaktiver Ester z.B. der Halogenide erfolgt analog.

Ein weiterer Gegenstand der vorliegenden Erfindung sind Arzneimittel mit cardiovaskulären Eigenschafrten, welche neben üblichen Füll- und Hilfsstoffen mindestens eine Verbindung der allgemeinen Formel I enthalten.

Die Dosierung beim Menschen dürfte bedingt durch die gute Verträglichkeit der Verbindungen und abhängig vom Schweregrad der Erkrankung bei etwa 20-250 mg bei oraler bzw. parenteraler einmaliger Verabreichung liegen.

Die erfindungsgemässen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Äthanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süssstoffe enthalten.

Das folgende Beispiel dient der näheren Erläuterung der Erfindung:

*Beispiel*

*1-Aminomethylcyclohexylmethan-sulfonsäure*

33,4 g (150 mMol) 1-Aminomethylcyclohexylmethyl-hydrogensulfat und 37,8 g (300 mMol) Natriumsulfit werden in 150 ml Wasser im Autoklaven 6 Stunden auf 160° C erhitzt. Nach dem Erkalten saugt man den erhaltenen Kristallbrei scharf ab, wäscht mit wenig Eiswasser nach und kristallisiert aus Wasser um. Man erhält 1-Aminomethylcyclohexylmethan-sulfonsäure als farblose Nadeln, die zwischen 390 und 410° C schmelzen.

In analoger Weise erhält man, ausgehend von 1-Aminomethylcyclopentylmethyl-hydrogensulfat bzw. 1-Aminomethylcycloheptyl-methylhydrogensulfat:

*1-Aminomethylcyclopentylmethan-sulfonsäure*
*1-Aminomethylcycloheptylmethan-sulfonsäure*

*Pharmakologische Vergleichsuntersuchung:*
*1-Aminomethylcyclohexylmethan-sulfonsäure/Procainamid.*

*Therapeutische Wirkung bei $CaCl_2$-induzierter Arrhythmie an der Ratte*
(Arrhythmie durch extrazelluläre Elektrolytstörung).

*Methode:* Durch intravenöse Infusion von Calciumchlorid-Lösung werden an narkotisierten Ratten Arrhythmien induziert, die nach Beendigung der Infusion über einen bestimmten Zeitraum anhalten [modifiziert nach Malinow, M.R., et al., *Arch. int. Pharmacodyn., 102,* 266 (1955)]. Diese arrhythmische Phase wird durch die prophylaktische Applikation von Antiarrhythmica aufgehoben. Die Wirkungen der erfindungsgemässen Substanz (I) werden mit denjenigen von Procainamid verglichen.

| Substanz | Arrhythmogene Menge $CaCl_2$-Lösg. (in mg/kg) | Substanz-Dosis zur Aufhebung der Arrhythmie (mg/kg i.v.) | LD50 (i.g.) mg/kg (Maus) |
|---|---|---|---|
| I (n = 5) | 278 ± 35 | 5 | >1600 |
| Procainamid | 193 ± 27 | 20 | 1200 |
| Kontrolle (NaCl) | 59 ± 10 | — | — |

Die erfindungsgemässen Verbindungen sind hinsichtlich Verträglichkeit und Potenz dem bekannten Antiarrhythmicum Procainamid überlegen.

## I. Patentansprüche für die Vertragsstaaten CH, DE, FR, GB, IT, LI

1. Aminosulfonsäuren der allgemeinen Formel I

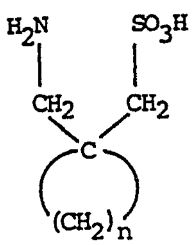

(I)

in welcher n die Zahlen 4, 5 oder 6 bedeutet.

2. 1-Aminomethylcyclohexylmethan-sulfonsäure.

3. Verfahren zur Herstellung von Verbindungen der allgemeinen Formel I

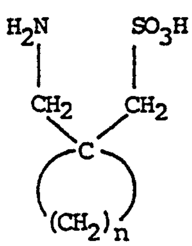

(I)

in welcher n die Zahlen 4, 5 oder 6 bedeutet, dadurch gekennzeichnet, dass man eine Verbindung der allgemeinen Formel II

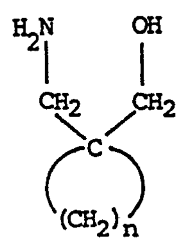

(II)

in welcher n die obengenannte Bedeutung hat, in an sich bekannter Weise zunächst in einen reaktiven Ester überführt und diesen mit einem löslichen anorganischen Sulfit umsetzt.

4. Verfahren gemäss Anspruch 3, dadurch gekennzeichnet, dass man als reaktiven Ester einen Schwefelsäurehalbester der allgemeinen Formel III

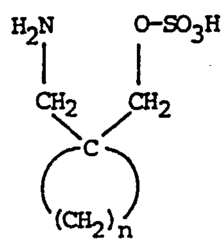

(III)

in welcher n die obengenannte Bedeutung hat, verwendet.

5. Arzneimittel, dadurch gekennzeichnet, dass sie neben üblichen Füll- und Hilfsstoffen mindestens eine Verbindung gemäss Anspruch 1 oder 2 enthalten.

## II. Patentansprüche für den Vertragsstaat: AT

1. Verfahren zur Herstellung von Verbindungen der allgemeinen Formel I

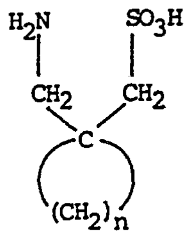

(I)

in welcher n die Zahlen 4, 5 oder 6 bedeutet, dadurch gekennzeichnet, dass man eine Verbindung der allgemeinen Formel II

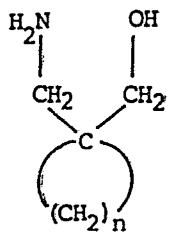

(II)

in welcher n die obengenannte Bedeutung hat, in an sich bekannter Weise zunächst in einen reaktiven Ester überführt und diesen mit einem löslichen anorganischen Sulfit umsetzt.

2. Verfahren gemäss Anspruch 1, dadurch gekennzeichnet, dass man als reaktiven Ester einen Schwefelsäurehalbester der allgemeinen Formel III

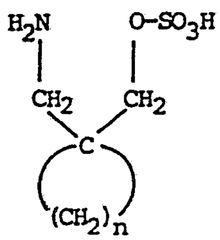

(III)

in welcher n die obengenannte Bedeutung hat, verwendet.

3. Verfahren gemäss Anspruch 1 und 2 zur Herstellung von 1-Aminomethylcyclohexylmethansulfonsäure.

## Claims for the contracting States CH, DE, FR, GB, IT, LI

1. Amino sulfonic acid of the general formula I

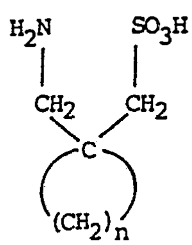

(I)

wherein n is the figures 4, 5 or 6.

2. Aminomethyl cyclohexylmethane-sulfonic acid.

3. Process for the manufacture of compounds of the general formula I

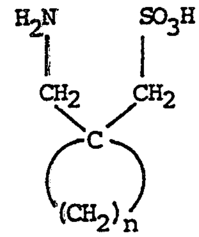

(I)

wherein n is the figures 4, 5 or 6, characterized by first transferring in a known manner a compound of the general formula II

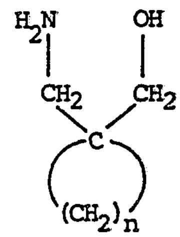

(II)

wherein n has the above meaning into a reactive ester, which is then reacted with a soluble anorganic sulfite.

4. Process according to claim 2, characterized by using as the reactive ester a semiester of sulfuric acid of the general formula III

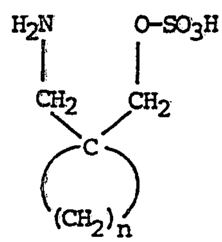

(III)

wherein n has the above meaning.

5. Pharmaceutical preparation, characterized by the contents of at least one compound according to claims 1 or 2 and usual fillers and adjuvants.

## Claims for the contracting State: AT

1. Process for the manufacture of compounds of the general formula I

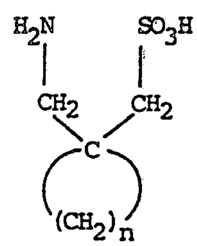

(I)

wherein n is the figures 4, 5 or 6, characterized by first transferring in a known manner a compound of the general formula II

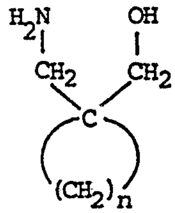

(II)

wherein n has the above meaning into a reactive ester, which is then reacted with a soluble anorganic sulfite.

2. Process according to claim 1, characterized by using as the reactive ester a semiester of sulfuric acid of the general formula III

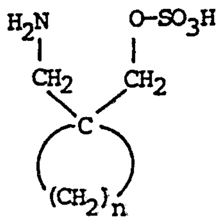

(III)

wherein n has the above meaning.

3. Process according to claims 1 and 2 for the manufacture of 1-aminomethylcyclohexylmethanesulfonic acid.

**Revendications pour les Etats contractants CH, DE, FR, GB, IT, LI**

1. Acides aminosulfoniques de formule générale I:

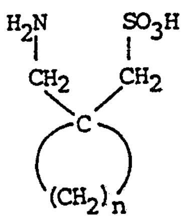

(I)

dans laquelle n est égal à 4, 5 ou 6.

2. Acide aminométhylcyclohexylméthanesulfonique.

3. Procédé de préparation de dérivés de formule générale I:

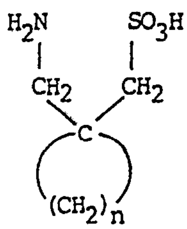

(I)

dans laquelle n est égal à 4, 5 ou 6, caractérisé en ce que l'on transforme un composé de formule générale II:

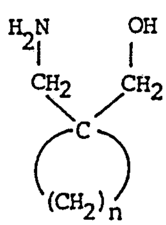

(II)

dans laquelle n présente la signification ci-dessus, tout d'abord d'une façon connue en soi en un ester réactif, et que l'on fait réagir celui-ci avec un sulfite minéral soluble.

4. Procédé selon la revendication 3, caractérisé en ce que l'on utilise comme ester réactif un semi-ester d'acide sulfurique de formule générale III:

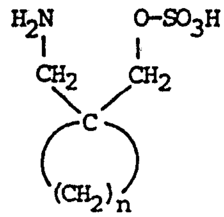

(III)

dans laquelle n présente la signification ci-dessus.

5. Médicaments, caractérisés en ce que, outre les charges et adjuvants usuels, ils contiennent au moins un composé selon la revendication 1 ou 2.

**Revendications pour l'Etat contractant: AT**

1. Procédé de préparation de composés de formule générale I:

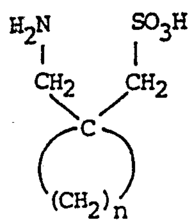

(I)

dans laquelle n est égal à 4, 5 ou 6, caractérisé en ce que l'on fait passer un composé de formule générale II:

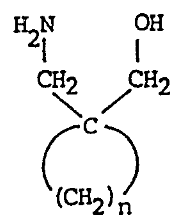

(II)

dans laquelle n présente la signification ci-dessus, d'une façon connue en soi tout d'abord en un ester réactif, et que l'on traite celui-ci par un sulfite organique soluble.

2. Procédé selon la revendication 1, caractérisé en ce que l'on emploie comme esters réactifs un semi-ester d'acide sulfurique de formule générale III:

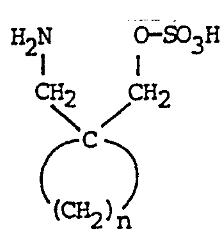

(III)

dans laquelle n possède la signification ci-dessus.

3. Composés selon les revendications 1 et 2 de préparation d'acide 1-aminométhylcyclohexylméthanesulfonique.